# FASCICULE DE BREVET EUROPEEN

(11) **EP 2 219 531 B1**
(45) Date de publication et mention de la délivrance du brevet: **09.11.2011**
(21) Numéro de dépôt: 08865661.6
(22) Date de dépôt: 12.12.2008
(51) Int. Cl.: A61B 17/068, A61F 2/00

(54) **APPAREIL DE POSE DE SPIRES DE SUTURE RESULTANT D'UN FIL METALLIQUE A MEMOIRE DE FORME**
VORRICHTUNG ZUR PLATZIERUNG VON STICHWICKLUNGEN AUS EINEM FORMSPEICHER-METALLFADEN
APPARATUS FOR PLACING STITCH TURNS RESULTING FROM A SHAPE-MEMORY METAL THREAD

(30) Priorité: 13.12.2007 FR 0759801
(43) Date de publication de la demande: 25.08.2010
(73) Titulaire: Microval, 43240 Saint Just Malmont (FR)
(72) Inventeur: PAIN, Bernard, F-43120 Monistrol sur Loire (FR); CUILLERON, Olivier, F-42270 Saint Priest en Jarez (FR)
(74) Mandataire: Thivillier, Patrick
(86) Numéro de dépôt international: PCT/FR2008/052287
(87) Numéro de publication internationale: WO 2009/081004

(56) Documents cités:
- WO-A-00/07506
- WO-A-2007/017562
- US-B1- 6 457 625
- US-B1- 6 699 255

## Description

L'invention se rattache au secteur technique des appareils pour la fixation et l'immobilisation d'une structure maillée faisant office de prothèse sur une partie anatomique.

Ces structures maillées faisant office de prothèses, sont parfaitement connues et peuvent, par exemple, être utilisées pour la région inguinale en étant destinées à la réparation de hernies inguinales par voie laparoscopique ou ouverte. L'immobilisation de ce type d'implant peut être effectuée par différents moyens, tels que fils de suture, agrafes, ...

L'invention concerne plus particulièrement un mode de fixation et d'immobilisation d'une structure maillée faisant office de prothèse, au moyen d'un élément réalisé à partir d'un fil préformé apte à :
- constituer au moins une spire à l'état repos, c'est-à-dire sans aucune sollicitation ;
- être développé linéairement après introduction dans un élément tubulaire ;
- reprendre sa forme initiale à la sortie du tube.

Avantageusement mais non limitativement, les fils sont réalisés dans un matériau à mémoire de forme. Une telle solution ressort de l'enseignement du document WO 2007/017562 dont le demandeur de la présente est également le titulaire.

A partir de cette conception de base, l'élément d'immobilisation de la structure maillée, se pose le problème de la mise en place d'un tel

A partir de cette conception de base, l'élément d'immobilisation de la structure maillée, se pose le problème de la mise en place d'un tel élément qui, à l'état repos, est constitué d'une spire, de sorte qu'il doit, préalablement à sa mise en place, être développé linéairement, puis reprendre sa forme initiale, en constituant au moins une spire réalisée dans une partie de l'épaisseur de la partie anatomique et au travers de plusieurs mailles de la structure pour assurer les liaisons.

Pour atteindre cet objectif, le document WO 2007/017562 enseigne de disposer plusieurs fils, en alignement dans un tube qui présente des moyens aptes à assurer l'extraction d'un fil au moment de son impaction dans la structure maillée. Cette solution n'est cependant pas totalement satisfaisante, étant donné qu'il est apparu une certaine difficulté pour la mise en place de plusieurs spires à l'état développé à l'intérieur du tube avec des risques de chevauchement, notamment au moment de la mise en place.

Il est également connu dans l'art antérieur d'utiliser des appareils de pose de spires selon le préambule de la revendication 1 tels que divulgués notamment dans les documents US 6,699,255, US 6,457,625 ou WO 00/07506.

L'invention s'est fixée pour but de remédier à ces inconvénients d'une manière simple, sûre, efficace et rationnelle.

Le problème que se propose de résoudre l'invention est de pouvoir assurer la mise en place d'un élément d'immobilisation de structure maillée, ledit élément constituant, à l'état repos c'est-à-dire sans aucune sollicitation, au moins une spire, avec pour objectif de pouvoir garnir un appareil de pose de plusieurs fils à l'état développé linéairement et extraire séparément chaque fil de l'appareil, en constituant au moins une spire réalisée dans une partie de l'épaisseur de la partie anatomique au travers de plusieurs mailles de la structure pour assurer une liaison.

Pour résoudre un tel problème, it a été conçu et mis au point un appareil de pose de spires, de suture résultant d'un fil métallique a mémoire

de forme, l'appareil se présente sous la forme d'un pistolet avec un corps, une poignée de préhension et un organe de commande, ledit corps présentant coaxialement une tige creuse de visée débordante.

Selon l'invention, la tige est équipée, à son extrémité libre, d'un barillet contenant les fils, ladite tige étant assujettie à des moyens aptes à permettre, sous l'action de l'organe de commande, le déplacement d'un moyen d'éjection d'un fil pour la formation d'une spire à la sortie du barillet puis, lors du relâchement dudit organe de commande, une rotation partielle dudit barillet pour positionner un autre fil en regard dudit moyen d'éjection.

Compte tenu de ces caractéristiques, il suffit donc d'exercer, avec la tige équipée du barillet, un effort d'appui au niveau des zones de fixation considérées et d'agir sur l'organe de commande pour l'éjection d'un fil et concomitamment la formation d'une spire au travers des mailles de la structure et de la partie anatomique correspondante.

Pour résoudre le problème posé d'assurer la commande du barillet, le barillet est monté en bout d'un axe fixe en translation et engagé à l'intérieur de la tige, l'autre extrémité dudit axe coopérant avec des moyens aptes à assurer sa rotation partielle et, par conséquent, celle du barillet.

Pour résoudre le problème posé d'assurer la rotation du barillet au fur et à mesure de la mise en place d'une spire, les moyens aptes à assurer la rotation partielle de l'axe et du barillet, sont constitués par une bague dentée tournante indexée angulairement sur l'axe et dont les dents présentent une extrémité profilée engagée dans un profil complémentaire d'une denture formée en bout d'une douille montée fixement dans le corps. Sur ladite douille, est monté, avec capacité de déplacement linéaire guidé et d'indexation en rotation, un manchon à l'intérieur duquel est montée la bague dentée à l'encontre d'un ressort, ledit manchon étant déplaçable coaxialement sous l'action de l'organe de commande.

Pour résoudre le problème posé d'assurer l'éjection du fil métallique, le manchon est monté avec capacité de déplacement coaxial à l'encontre d'un ressort de rappel et est solidaire d'un tube monté coulissant sur l'axe, ledit tube étant assujetti au moyen d'éjection en vue de son déplacement en alignement avec un logement du barillet.

Pour résoudre le problème posé d'assurer l'accouplement du barillet, le moyen d'éjection est une aiguille accouplée à l'une de ses extrémités à un guide solidaire du tube coulissant et guidée, à son autre extrémité, dans un embout solidaire de la tige.

Dans une forme de réalisation, l'extrémité de l'axe présente une portée méplate coopérant avec une portée coaxiale interne complémentaire du barillet.

Selon une autre caractéristique, le barillet est monté dans un fourreau accouplé, d'une manière démontable, à l'embout, ledit fourreau recevant un élément de protection et d'appui présentant un orifice débouchant en communication avec le logement du barillet disposé en regard de l'aiguille d'éjection.

L'organe de commande est une détente articulée sur le corps dont une extrémité présente une fourchette coopérant avec un bossage du manchon.

L'invention est exposée ci-après plus en détail à l'aide des figures des dessins annexés dans lesquels :

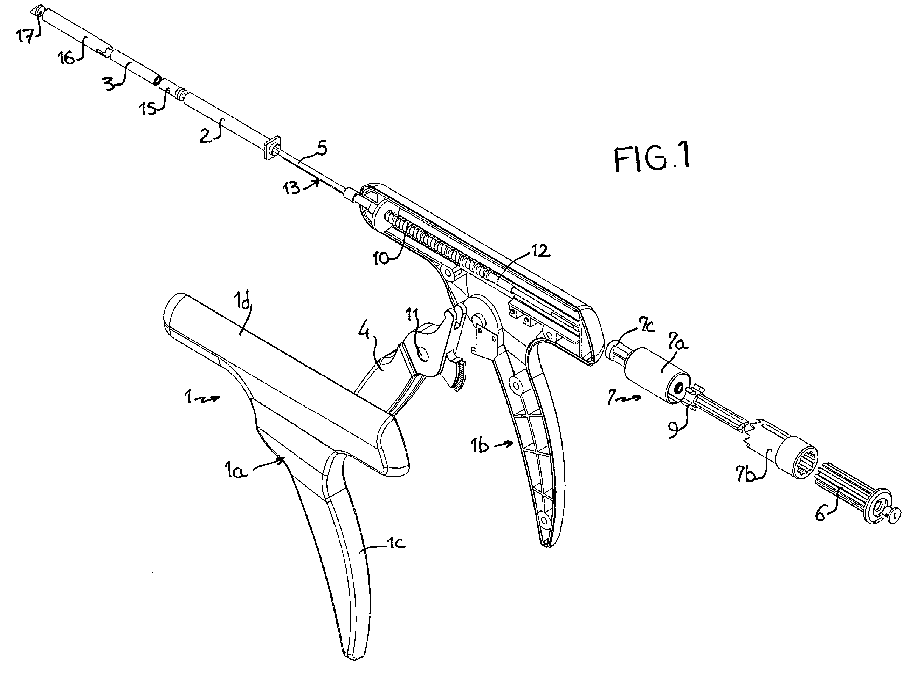

- la figure 1 est une vue en perspective des principaux éléments de l'appareil avant montage ;

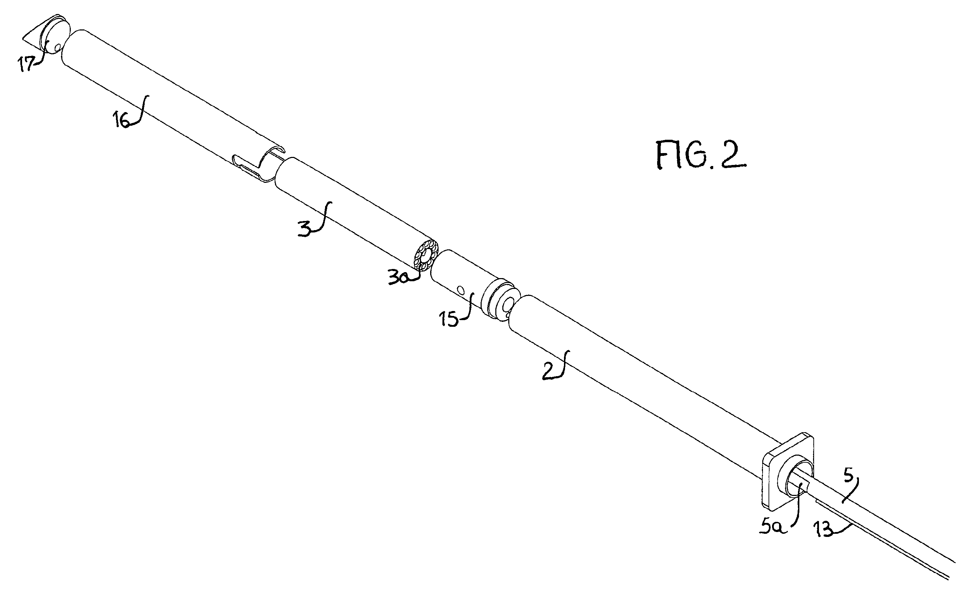

- la figure 2 est une vue en perspective à plus grande échelle montrant le montage des éléments relatifs à l'accouplement du barillet ;

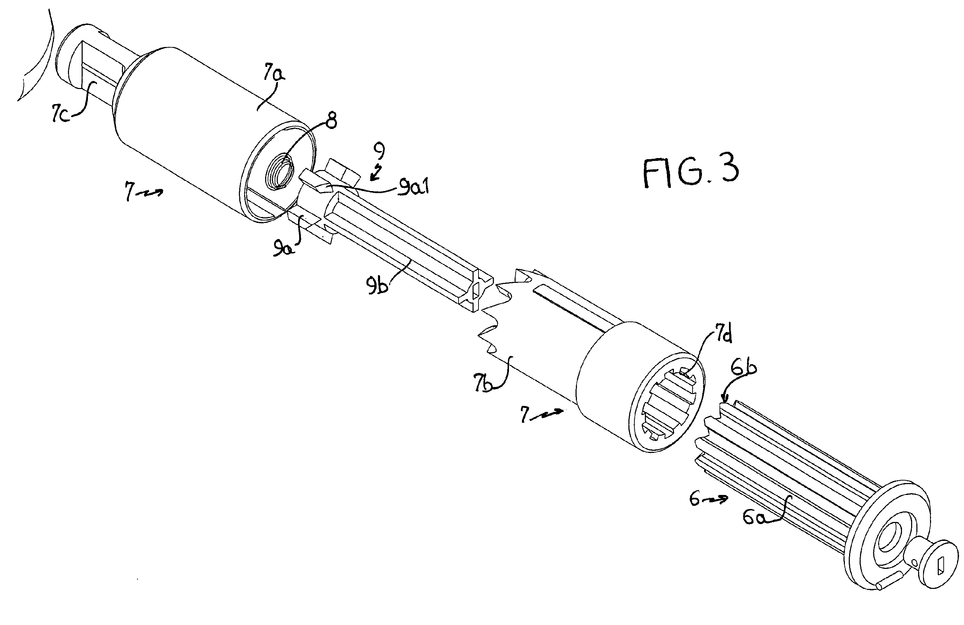

- la figure 3 est une vue en perspective, à plus grande échelle avant montage, des principaux éléments montrant une forme de réalisation du moyen d'entraînement en rotation du barillet ;

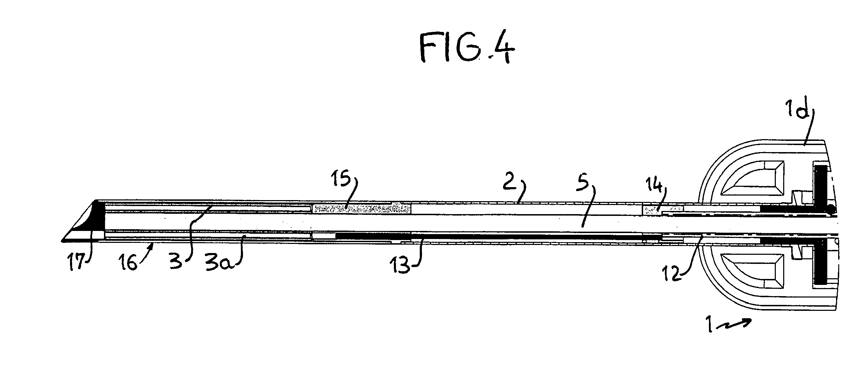

- la figure 4 est une vue en coupe longitudinale, au niveau de l'extrémité de la tige ;

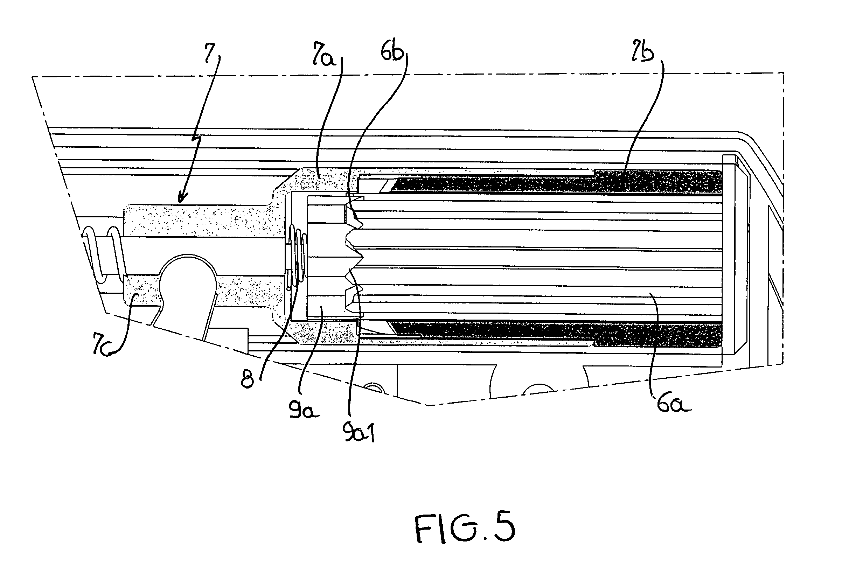

- la figure 5 est une vue en coupe longitudinale au niveau des agencements d'entraînement en rotation du barillet ;

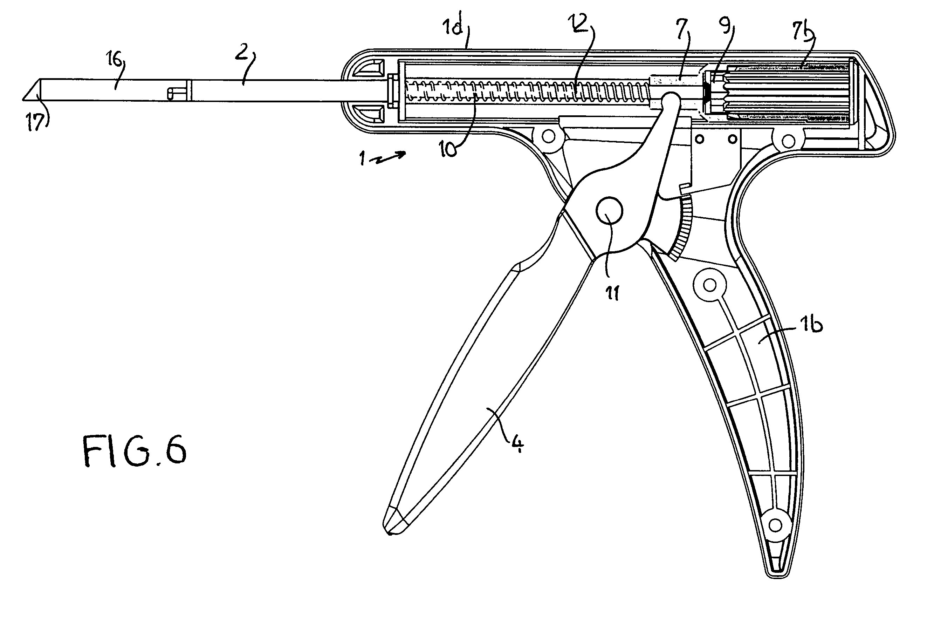

- la figure 6 est une vue en coupe longitudinale partielle de l'ensemble de l'appareil après montage des éléments.

On rappelle, comme il ressort de l'enseignement du document WO 2007/017562, que l'élément d'immobilisation de la structure maillée prothétique, est réalisé à partir d'un fil conformé pour constituer, à l'état repos, au moins une spire. Le fil est réalisé dans un matériau sélectionné pour être développé linéairement, après introduction dans un organe de section tubulaire, et reprendre sa forme de spire à la sortie dudit organe. Avantageusement, le fil peut être réalisé dans un matériau à mémoire de forme.

Selon l'invention, l'appareil, pour la mise en place de ce type de spire, présente la forme générale d'un pistolet. Le pistolet présente un corps (1) réalisé, par exemple, à partir de deux demi-coquilles en matière plastique (1a) et (1b) formant une poignée de préhension (1c) établie angulairement à partir d'un canon (1d) de forme générale tubulaire. Le canon (1d) est solidaire d'une tige de visée (2) au bout de laquelle est montée, selon une caractéristique à la base de l'invention, un barillet (3). Les logements (3a) du barillet (3) sont destinés à recevoir chacun un fil pour la formation d'une spire dans les conditions indiquées précédemment.

Comme il sera indiqué dans la suite de la description, la tige (2) est assujettie à des moyens aptes à permettre, sous l'action d'un organe de commande (4), le déplacement d'un moyen d'éjection d'un fil pour la formation d'une spire à la sortie du barillet (3), puis, à chaque impulsion donnée sur l'organe de commande (4), une rotation partielle dudit barillet (3) pour positionner un autre de ses logements (3a) contenant un fil, en regard dudit moyen d'éjection.

Dans la forme de réalisation illustrée aux figures des dessins, le barillet (3) est fixé, avec capacité de démontage, un bout d'un axe fixe en translation (5) monté à l'intérieur du corps (1d) et engagé dans la tige (2). Par exemple, l'extrémité de l'axe (5) présente une portée méplate (5a) coopérant avec une portée coaxiale, de section complémentaire, que présente le barillet (3). L'autre extrémité (5b) de l'axe est asservie à des moyens aptes à assurer sa rotation partielle et, de manière concomitante, celle du barillet (3). L'autre extrémité de l'axe (5) est accouplée à une douille (6) montée fixement à l'intérieur du corps (1), notamment à l'extrémité de la partie (1d) faisant office de canon, à l'opposé de celle recevant la tige (2).

La douille (6) reçoit, avec capacité de déplacement linéaire guidé et d'indexation en rotation, un manchon (7). Par exemple, la douille (6) peut présenter périphériquement une série de cannelures (6a) coopérant avec des cannelures complémentaires (7d) formées dans l'alésage du manchon (7). A l'intérieur du manchon (7), est montée tournante, à l'encontre d'un ressort (8), une bague dentée (9). Par exemple, la bague (9) présente une tête (9a) avec, périphériquement, une série de dents (9a1). Cette tête est prolongée par une portée (9b) engagée librement dans l'alésage de la douille (6). Les extrémités des dents (9a1) de la bague (9) présentent un profil faisant office de rampe, coopérant avec un profil complémentaire (6b) d'une denture formée en bout de la douille (6).

Le manchon (7) est déplaçable coaxialement sous l'action de l'organe de commande (4), à l'encontre d'un ressort de rappel (10). Dans l'exemple illustré, le manchon (7) est réalisé en deux parties (7a) et (7b), assemblées entre elles, pour constituer un ensemble unitaire. Bien évidemment, une conception monobloc (7) du manchon peut être prévue. L'organe de commande (4), sous forme sensiblement d'une détente, est articulé sur un axe transversal (11) et présente une fourchette (4a) coopérant, par exemple, avec un décrochement (7c) du manchon (7) pour son entraînement en translation, sous un effet de pivotement par rapport à l'axe (11).

Le manchon (7), par exemple la partie (7a), est solidaire d'un tube (12) monté coulissement sur l'axe (5) à l'intérieur de la tige (2). Ce tube (12) est assujetti au moyen d'éjection en vue de son déplacement en alignement avec un logement (3a) du barillet (3), après rotation de ce dernier. Dans la forme de réalisation illustrée, le moyen d'éjection est constitué par une aiguille (13) accouplée à l'une de ses extrémités à un guide (14) solidaire du tube coulissant (12). A son autre extrémité, l'aiguille (13) est engagée librement dans un embout (15), solidaire de la tige (2). Cet embout (15) fait également office de palier à l'axe (5) de commande en rotation du barillet (3).

Dans la forme de réalisation illustrée, le barillet (3) est monté à l'intérieur d'un fourreau (16) accouplé, de manière démontable, à l'embout (15). Par exemple, le fourreau (16) est emmanché sur une portée cylindrique de l'embout (15). A l'extrémité libre du fourreau (16), est monté un élément de protection et d'appui (17) présentant un orifice débouchant (17a), en communication avec le logement considéré (3a) du barillet (3) disposé en regard de l'aiguille d'éjection (13).

Le fourreau (16), recevant le barillet (3), peut présenter des moyens d'indication visuelle de l'état de chargement dudit barillet.

Le fonctionnement de l'appareil est particulièrement simple et efficace, et d'utilisation aisée pour les praticiens.

Le barillet (3) est préalablement garni, dans chaque compartiment (3a), d'un fil constituant, à l'état repos, la spire de suture. A titre indicatif, le barillet peut présenter dix logements. Ainsi garni, le barillet (3) est accouplé en bout de l'axe (5) en butée contre l'embout (15). Après mise en place du fourreau (16) sur l'embout (15), en alignement avec la tige (2) et l'élément de protection (17), l'appareil est prêt à être utilisé.

Le praticien dispose la tige de visée (2) équipée comme indiqué, du barillet (3) du fourreau (16) et de l'élément (17), en appui sur la partie de la structure maillée qui doit être fixée à une partie de l'anatomie correspondante, au moyen de la spire. Dans cette position, un appui sur la gâchette (4) a pour effet de commander en translation le fourreau (7) équipé du tube (12) et, par conséquent, d'assurer le déplacement de l'aiguille d'éjection (13) qui va pousser le fil disposé dans le logement correspondant du barillet. Le fil poussé par l'aiguille (13), sort par l'orifice (17a) de l'élément de protection (17) pour permettre son impaction au travers des mailles de la plaque prothétique et dans l'épaisseur de la partie anatomique correspondante. Comme indiqué, sous l'effet de la libération du fil, il y a formation d'au moins une spire assurant l'immobilisation et la fixation de la partie correspondante de la structure maillée.

D'une manière importante, sous l'effet du déplacement précité, la bague dentée (9), par son profil de denture échappe le profil complémentaire (6b) et, sous l'effet de poussée du ressort (8), retombe dans un profil de denture adjacent provoquant, d'une manière concomitante, à la façon d'un cliquet, l'entraînement en rotation de la dite bague et, par conséquent, de l'axe (5) recevant au bout le barillet (3). Il en résulte une rotation partielle du barillet (3) pour la mise en correspondance d'un autre logement en regard de l'aiguille d'éjection (13). L'appareil est par conséquent prêt pour la mise en place d'une autre spire de suture.

La bague (9), notamment par la portée (9b), est indexée angulairement sur l'axe (5), de sorte que la rotation partielle de cette bague entraîne la rotation partielle de l'axe (5).

Compte tenu de ces caractéristiques, il est possible d'utiliser, avec le même appareil, plusieurs barillets prêts et garnis de fils de suture.

D'une manière préférée, l'appareil est destiné à être jeté après chaque utilisation.

De même, sans pour cela sortir du cadre de l'invention, l'appareil peut être utilisé pour la mise en place et la fixation de tout type d'implant de suture de forme générale allongée, notamment filiforme.

Les avantages ressortent bien de la description, en particulier on souligne et on rappelle :
- l'utilisation d'une spire préformée
- l'utilisation d'un barillet rotatif permettant la mise en place de plusieurs spires, sans être obligé de recharger ;
- la facilité d'utilisation de l'appareil, étant donné qu'il suffit d'appuyer et de relâcher la détente ;
- la simplicité de réalisation ;
- la fiabilité de fonctionnement.

## Revendications

1. Appareil de pose de spires de suture résultant d'un fil métallique à mémoire de forme, l'appareil se présente sous la forme d'un pistolet avec un corps (1), une poignée de préhension (1c) et un organe de commande (4), ledit corps (1) présentant coaxialement une tige creuse de visée débordante (2) équipée, à son extrémité libre, du barillet (3) contenant les fils, ladite tige (2) étant assujettie à des moyens aptes à permettre, sous l'action de l'organe de commande (4), le déplacement d'un moyen d'éjection (13) d'un fil pour la formation d'une spire à à sortie du barillet (3), **caractérisé en ce que** le barillet (3) est monté en bout d'un axe (5) fixe en translation et engagé à l'intérieur de la tige (2), l'autre extrémité dudit axe (5) coopérant avec des moyens aptes à assurer sa rotation partielle et celle dudit barillet (3), lors du relâchement dudit organe de commande (4), pour positionner un autre fil en regard dudit moyen d'éjection.

2. Appareil selon la revendication 1, **caractérisé en ce que** les moyens aptes à assurer la rotation partielle de l'axe (5) et du barillet (3), sont constitués par une bague dentée tournante (9) indexée angulairement sur l'axe (5) et dont les dents présentent une extrémité profilée (9a1) engagée dans un profil complémentaire d'une denture (6b) formée en bout d'une douille (6) montée fixement dans le corps (1), sur ladite douille (6), est monté, avec capacité de déplacement linéaire guidé et d'indexation en rotation, un manchon (7) à l'intérieur duquel est montée la bague dentée (9) à l'encontre d'un ressort (8), ledit manchon (7) étant déplaçable coaxialement sous l'action de l'organe de commande (4).

3. Appareil selon la revendication 2, **caractérisé en ce que** le manchon (7) est monté avec capacité de déplacement coaxial à l'encontre d'un ressort de rappel (10).

4. Appareil selon l'une quelconque des revendications 2 à 3, **caractérisé en ce que** le manchon (7) est solidaire d'un tube (12) monté coulissant sur l'axe (5), ledit tube (12) étant assujetti au moyen d'éjection (13) en vue de son déplacement en alignement avec un logement du barillet.

5. Appareil selon l'une quelconque des revendications 1 à 4, **caractérisé en ce que** le moyen d'éjection (13) est une aiguille accouplée à l'une de ses extrémités à un guide (14) solidaire du tube coulissant (12) et guidée, à son autre extrémité, dans un embout (15) solidaire de la tige (2).

6. Appareil selon la revendication 1, **caractérisé en ce que** l'extrémité de l'axe (5) présente une portée méplate coopérant avec une portée coaxiale interne complémentaire du barillet (3).

7. Appareil selon l'une quelconque des revendications 1 à 6, **caractérisé en ce que** le barillet (3) est monté dans un fourreau (16) accouplé, d'une manière démontable, à l'embout (15), ledit fourreau (16) recevant un élément de protection et d'appui (17) présentant un orifice débouchant (17a) en communication avec le logement (3 a) du barillet (3) disposé en regard de l'aiguille d'éjection (13).

8. Appareil selon l'une quelconque des revendications 1 à 7, **caractérisé en ce que** l'organe de commande (4) est une détente articulée sur le corps (1) dont une extrémité présente une fourchette coopérant avec un bossage du manchon (7).

9. Appareil selon la revendication 8, **caractérisé en ce que** le fourreau (16) recevant le barillet (3) présente des moyens d'indication visuelle de l'état de chargement du barillet.

## Claims

1. Appliance for the placement of stitch turns resulting from a shape-memory metal thread, the appliance coming in the form of a gun with a body (1), a grip handle (1c) and a control member (4), said body (1) having coaxially a hollow projecting sight rod (2) fitted, at its free end, with a barrel (3) containing the threads, said rod (2) being subjected to means capable, under the action of the control member (4), of allowing a thread ejection means (13) to move in order to form a turn at the output from the barrel (3), **characterised in that** the barrel (3) is mounted at the end of a shaft (5) fixed in translatory motion and engaged inside the rod (2), the other end of said shaft (5) engaging with means capable of partially rotating it and said barrel (3), when said control member (4) is released, in order to position another thread opposite said ejection means.

2. Appliance as claimed in claim 1, **characterised in that** the means capable of partially rotating the shaft (5) and the barrel (3), are constituted by a rotating toothed ring (9) angularly indexed on the shaft (5) and whereof the teeth have a shaped end (9a1) engaged in a complementary profile of a toothing (6b) formed at the end of a bush (6) securely mounted in the body (1); on said bush (6), is mounted, with guided linear movement and indexation in rotation capability, a sleeve (7) inside which the toothed ring (9) is mounted against a spring (8), said sleeve (7) being movable coaxially under the action of the control member (4).

3. Appliance as claimed in claim 2, **characterised in that** the sleeve (7) is mounted with coaxial movement capability against a return spring (10).

4. Appliance as claimed in any one of claims 2 to 3, **characterised in that** the sleeve (7) is secured to a tube (12) mounted sliding on the shaft (5), said tube (12) being subject to the ejection means (13) so that it can be moved in alignment with a housing of the barrel.

5. Appliance as claimed in any one of claims 1 to 4, **characterised in that** the ejection means (13) is a needle coupled at one of its ends to a guide (14) secured to the sliding tube (12) and guided, at its other end, in an end piece (15) secured to the rod (2).

6. Appliance as claimed in claim 1, **characterised in that** the end of the shaft (5) has a flat bearing surface engaging with a complementary internal coaxial bearing surface of the barrel (3).

7. Appliance as claimed in any one of claims 1 to 6, **characterised in that** the barrel (3) is mounted in a sheath (16) coupled, in a removable manner, to the end piece (15), said sheath (16) accommodating a protective and support element (17) having a through orifice (17a) in communication with the housing (3a) of the barrel (3) placed opposite the ejection needle (13),

8. Appliance as claimed in any one of claims 1 to 7, **characterised in that** the control member (4) is a trigger articulated on the body (1) whereof one end has a fork engaging with a projection of the sleeve (7).

9. Appliance as claimed in claim 8, **characterised in that** the sheath (16) accommodating the barrel (3) has means for the visual display of the load charge status.

## Patentansprüche

1. Gerät zum Anbringen von Nahtwindungen aus einem Formgedächtnisdraht, das die Form einer Pistole aufweist mit einem Körper (1), einem Griff (1c) und einem Betätigungsorgan (4), wobei der Körper (1) koaxial eine vorstehende hohle Zielstange (2) aufweist, die an ihrem freien Ende mit einer die Drähte enthaltenden Trommel (3) ausgestattet ist, während die besagte Stange (2) von Einrichtungen abhängig ist, die geeignet sind, unter Einwirkung des Betätigungsorgans (4) die Verlagerung einer Auswurfeinrichtung (13) eines Drahtes für die Bildung einer Windung am Ausgang der Trommel (3) zu ermöglichen, **dadurch gekennzeichnet, dass** die Trommel (3) am Ende einer verschiebfesten und in die Stange (2) eingeführten Achse (5) angebracht ist, während das andere Ende der Achse (5) mit Einrichtungen zusammenwirkt, die geeignet sind, beim Loslassen des besagten Betätigungsorgans (4) seine teilweise Drehung und diejenige der Trommel (3) zu gewährleisten, um einen anderen Draht gegenüber der besagten Auswurfeinrichtung in Position zu bringen.

2. Gerät nach Anspruch 1, **dadurch gekennzeichnet, dass** die Einrichtungen, die geeignet sind, für die teilweise Drehung der Achse (5) und der Trommel (3) zu sorgen, aus einem drehbaren gezahnten Ring (9) bestehen, der an der Achse (5) winkelindexiert ist und dessen Zähne ein profiliertes Ende (9a1) aufweisen, das in ein ergänzendes Profil einer Zahnung (6b) am Ende einer ortsfest im Körper (1) angebrachten Hülse (6) eingeführt wird; auf der besagten Hülse (6) ist eine linear geführt verschiebbare und rotationsindexierte Muffe (7) angebracht, in der der gezahnte Ring (9) gegenüber einer Feder (8) installiert ist, wobei die besagte Muffe (7) unter Einwirkung des Betätigungsorgans (4) koaxial verschiebbar ist.

3. Gerät nach Anspruch 2, **dadurch gekennzeichnet, dass** die Muffe (7) gegenüber einer Rückholfeder (10) koaxial verschiebbar angebracht ist.

4. Gerät nach einem der Ansprüche 2 - 3, **dadurch gekennzeichnet, dass** die Muffe (7) mit einem gleitend auf der Achse (5) angebrachten Rohr (12) fest verbunden ist, wobei das Rohr (12) zwecks seiner Verschiebung in Ausrichtung zu einem Lager der Trommel von der Auswurfeinrichtung (13) abhängig ist.

5. Gerät nach einem der Ansprüche 1 - 4, **dadurch gekennzeichnet, dass** es sich bei der Auswurfeinrichtung (13) um eine Nadel handelt, die an einem ihrer Enden mit einer Führung (14) gekoppelt ist, die mit dem Gleitrohr (12) fest verbunden ist und an ihrem anderen Ende in einem mit der Stange (2) fest verbundenen Ansatzstück (15) geführt wird.

6. Gerät nach Anspruch 1, **dadurch gekennzeichnet, dass** das Ende der Achse (5) eine abgeflachte Auflagefläche aufweist, die mit einer internen koaxialen Auflagefläche komplementär zur Trommel (3) zusammenwirkt.

7. Gerät nach einem der Ansprüche 1 - 6, **dadurch gekennzeichnet, dass** die Trommel (3) in einer Hülle (16) angebracht ist, die demontierbar mit dem Ansatzstück (15) gekoppelt ist, wobei die Hülle (16) ein Schutz- und Auflageelement (17) aufnimmt, das eine ausführende Mündung (17a) aufweist, die mit dem Lager (3a) der der Auswurfnadel (13) zugewandten Trommel (3) in Verbindung steht.

8. Gerät nach einem der Ansprüche 1 - 7, **dadurch gekennzeichnet, dass** das Betätigungsorgan (4) ein am Körper (1) angelenkter Abzug ist, von dem ein Ende eine Gabel aufweist, die mit einer Erhebung der Muffe (7) zusammenwirkt.

9. Gerät nach Anspruch 8, **dadurch gekennzeichnet, dass** die die Trommel (3) aufnehmende Hülle (16) Einrichtungen für die optische Anzeige des Ladezustands der Trommel aufweist.
